(19) Europäisches Patentamt
European Patent Office
Office européen des brevets

(11) **EP 4 481 026 A1**

(12) **EUROPEAN PATENT APPLICATION**

(43) Date of publication:
**25.12.2024  Bulletin 2024/52**

(21) Application number: **23180609.2**

(22) Date of filing: **21.06.2023**

(51) International Patent Classification (IPC):
***C11D 3/38*** *(2006.01)*   ***C11D 3/386*** *(2006.01)*
***C11D 11/00*** *(2006.01)*   ***C12N 9/88*** *(2006.01)*

(52) Cooperative Patent Classification (CPC):
**C11D 3/381; C11D 3/38636; C12N 9/88;
C12Y 402/02003; C12Y 402/02011;** C11D 2111/12

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **The Procter & Gamble Company
Cincinnati, OH 45202 (US)**

(72) Inventors:
• **LANT, Neil Joseph
  Newcastle upon Tyne, NE12 9TS (GB)**
• **LATIMER, Katherine Esther
  Newcastle upon Tyne, NE12 9TS (GB)**

(74) Representative: **P&G Patent Belgium UK
N.V. Procter & Gamble Services Company S.A.
Temselaan 100
1853 Strombeek-Bever (BE)**

Remarks:
The complete document including Reference
Table(s) and the Sequence Listing(s) can be
downloaded from the EPO website

(54)   **DETERGENT COMPOSITIONS CONTAINING ENZYMES**

(57)   Detergent compositions particularly for laundry, comprising alginate lyase enzyme, and *Bacillus* spores. The compositions and methods are particularly good for improved stain removal from a fabric.

EP 4 481 026 A1

**Description**

REFERENCE TO A SEQUENCE LISTING

This application contains a Sequence Listing in computer readable form. The computer readable form is incorporated herein by reference.

FIELD OF THE INVENTION

The present invention relates to detergent compositions, particularly for laundry, comprising alginate lyase enzymes and *Bacillus* spores. The invention also relates to methods of cleaning using the detergent compositions.

BACKGROUND OF THE INVENTION

In cleaning applications, particularly for laundry, stain removal is a continuing problem. There are many cleaning technologies aimed at mitigating such problem however, it is a constant challenge to provide improved efficacy and especially in an environmentally favorable manner. In automatic washing machines these problems are compounded by the increased use of low wash temperatures (e.g., cold water) and shorter washing cycles which reduce stain/soil removal efficacy of detergent compositions.

Thus, it is an object of the present invention to provide a detergent composition, particularly for laundry which can be used in a washing process, even at low temperatures and short wash times, which will provide improved stain removal, in particular removal of protein-containing stains, more in particular sugary/fatty protein-containing stains, such as milk chocolate-based beverage or dessert stains.

SUMMARY OF THE INVENTION

The present invention provides a detergent composition, particularly for laundry, comprising (a) from 0.00005 to 5 wt% alginate lyase enzyme (active enzyme protein) and (b) *Bacillus* spores. Preferably the composition also comprises from 1 to 60 wt% anionic surfactant.

The invention also provides a method of treating a fabric, the method comprising contacting a fabric with an aqueous wash liquor comprising an alginate lyase enzyme and *Bacillus* spores, and optionally an anionic surfactant.

Preferably the aqueous wash liquor comprises anionic surfactant in an amount from 0.05g/l to 5g/l, preferably from 0.01g/l to 3g/l.

The fabric is typically contacted with the aqueous wash liquor at a temperature of 60°C or less, preferably at a temperature of 40°C or less or 35°C or less, most preferably at a temperature of 30°C or less or even 25°C or less; and (iii) optionally rinsing the surface. The compositions and methods herein are particularly useful for treating any fabric surface, synthetic or natural, including cotton, wool, silk, polyester, nylon, elastane or mixed fabric, such as polycotton.

The invention also relates to the use of a composition or method as described above for: improving stain removal. The composition and method described herein may also give rise to biofilm-disrupting effects.

DETAILED DESCRIPTION OF THE INVENTION

Definitions

Parent or Parent enzyme: The term "parent" or "parent enzyme" means an enzyme to which an alteration is made to produce the enzyme variants. The parent may be a naturally occurring (wild-type) polypeptide or a variant thereof. For example, the alginate lyase parent may be any of SEQ ID Nos: 1, 2, 3, 4, 5, 6 or 7 listed herein.

Sequence Identity: The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

Alternatively, the parameters used may be gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\text{(Identical Deoxyribonucleotides} \times 100)/(\text{Length of Alignment} - \text{Total Number of Gaps in Alignment)}$$

Variant: The term "variant" means a polypeptide having enzyme activity comprising an alteration/mutation, i.e., a substitution, insertion, and/or deletion, at one or more (e.g. several) positions relative to the parent. A substitution means a replacement of an amino acid occupying a position with a different amino acid; a deletion means removal of an amino acid occupying a position; and an insertion means adding 1-3 amino acids adjacent to and immediately following an amino acid occupying a position.

Wild-Type Enzyme: The term "wild-type" enzyme means an enzyme expressed by a naturally occurring microorganism, such as a bacterium, algae, yeast, or filamentous fungus found in nature.

Detergent Composition

The detergent composition comprises (a) from 0.00005 to 5 wt% alginate lyase enzyme (active enzyme protein) and (b) *Bacillus* spores. The detergent composition optionally comprises cleaning adjunct.

The detergent composition of the present invention is preferably a laundry detergent. The composition may be in the form of a composition for use in a main wash step or as pre-treatment or rinse-added cleaning composition for consumer or institutional use.

Alginate Lyase Enzyme

The alginate lyase is preferably microbial in origin, preferably bacterial or algal (for example from brown seaweed *(Phaeophyceae),* such as *Ascophyllum, Laminara, Macrocystis*), most preferably bacterial. The alginate lyase enzyme may be obtained from *Aeromonas sp., Azotobacter sp., Bacillus sp., Flavobacterium sp., Klebsiella sp., Pseudomonas sp., Sphingomonas sp., Vibrio sp., Zobellia galactanivorans,* most preferably *Flavobacterium sp.*

Preferably the alginate lyase enzyme comprises alginate lyase selected from: alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 1; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 3; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 75%, or at least 80%, or at least 85%, or at least 90%, or at least 95%, or at least 96%, or at least 97%, or at least 98%, or at least 99%, or 100%, sequence identity to SEQ ID NO: 7; or mixtures thereof. Preferred alginate lyase enzyme comprises alginate lyase enzyme corresponding to the wild-type or preferably a variant of the wild-type of any one of SEQ ID NOs: 1, 2, 3, 4, 5, 6 or 7 listed herein, or mixtures thereof. SEQ ID NOs 6 and 7 and variants thereof, and mixtures thereof are particularly preferred.

When the alginate lyase enzyme is a variant of a parent amino acid sequence, the parent alginate lyase enzyme preferably has a sequence identity to the polypeptide of one or more of SEQ ID NOs: 1, 2, 3, 4, 5, 6 or 7 of at least 50 % or at least 60%, or at least 70% or at least 80%, such as at least 85%, at least 90%, e.g. at least 92%, at least 93%, at least 94%, at least 95%, at least 96%, at least 97%, at least 98%, at least 99, or 100%, which has alginate lyase enzyme activity. It may be preferred for the variant amino acid sequence to differ from the parent alginate lyase by no more than fifteen, or no more than ten amino acids, or no more than five, or four or three or two or one amino acid(s) from the polypeptide of one or more of SEQ ID NOs: 1, 2, 3, 4, 5, 6 or 7.

When the alginate lyase enzyme is a variant of a parent amino acid sequence, the parent may be obtained from a microorganism of any genus. For purposes of the present invention, the term "obtained from" as used herein in connection with a given source shall mean that the parent encoded by a polynucleotide is produced by the source or by a cell in which the polynucleotide from the source has been inserted. In one aspect, the parent is secreted extracellularly. Variants may be prepared using any mutagenesis procedure known in the art, such as site-directed mutagenesis, synthetic gene construction, semi-synthetic gene construction, random mutagenesis, shuffling, etc.

The alginate lyase may be from any polysaccharide lyase (PL) family including PL5, PL6, PL7, PL14, PL15, PL17, PL18, PL32, PL34, and PL36. Preferably the alginate lyase is from PL family 7.

The alginate lyase enzyme may have activity towards poly(beta-D-mannuronate) (polyM activity) and/or activity towards poly(alpha-L-guluronate) (polyG activity). The alginate lyase enzyme may be incorporated into the cleaning compositions and methods of the invention in the form of a substantially pure enzyme.

The alginate lyase enzyme may be in the form of a liquid or a dry composition. For instance, the composition may be in the form of a granulate or a microgranulate. The alginate lyase enzyme may be stabilized including by encapsulation, in accordance with methods known in the art.

The alginate lyase enzyme is present in the composition in an amount from 0.00005 to 5 wt% active enzyme protein, preferably from 0.0001 to 2 wt% active protein or from 0.0005 or from 0.001 to 1 wt% active protein, or to 0.5 wt% or to 0.1 wt% or to 0.05 wt% active enzyme protein.

Preferably the alginate lyase enzyme is present in the aqueous wash liquor in an amount of from 0.01ppm to 1000 ppm of the enzyme, or from 0.05 or from 0.1ppm to 750 or 500ppm.

Bacterial spores

Although bacterial spores can be present on surfaces, the composition of the invention involves the intentional addition of bacterial spores to the composition in an amount capable of providing a consumer noticeable cleaning benefit. Preferably, the composition of the invention requires the intentional addition of from about $1 \times 10^2$ to about $1 \times 10^{11}$ CFU/g of bacterial spores.

The bacterial spores are fabric-substantive. The bacterial spores of the composition of the invention can germinate on fabrics. The spores can be activated by heat, for example, heat generated during use of the fabric or by the heat provided in the washing machine or in the dryer. The spores can germinate when the fabrics are stored and/or used. Malodor precursors can be used by the bacteria produced by the spores as nutrients promoting germination. Spores can germinate after the fabrics are left in the humid environment.

The spores have the ability to germinate and to form cells after the fabric is subjected to the laundry process. The spores can be delivered in liquid or solid form. Preferably, the spores are in solid form.

Some gram-positive bacteria have a two-stage lifecycle in which growing bacteria under certain conditions such as in response to nutritional deprivation can undergo an elaborate developmental program leading to spores or endospores formation. The bacterial spores are protected by a coat consisting of about 60 different proteins assembled as a biochemically complex structure with intriguing morphological and mechanical properties. The protein coat is considered a static structure that provides rigidity and mainly acting as a sieve to exclude exogenous large toxic molecules, such as lytic enzymes. Spores play critical roles in long term survival of the species because they are highly resistant to extreme environmental conditions. Spores are also capable of remaining metabolically dormant for years. Methods for obtaining bacterial spores from vegetative cells are well known in the field. In some examples, vegetative bacterial cells are grown in liquid medium. Beginning in the late logarithmic growth phase or early stationary growth phase, the bacteria may begin to sporulate. When the bacteria have finished sporulating, the spores may be obtained from the medium, by using centrifugation for example. Various methods may be used to kill or remove any remaining vegetative cells. Various methods may be used to purify the spores from cellular debris and/or other materials or substances. Bacterial spores may be differentiated from vegetative cells using a variety of techniques, like phase-contrast microscopy, automated scanning microscopy, high resolution atomic force microscopy or tolerance to heat, for example.

Because bacterial spores are generally environmentally-tolerant structures that are metabolically inert or dormant, they are readily chosen to be used in commercial microbial products. Despite their ruggedness and extreme longevity, spores can rapidly respond to the presence of small specific molecules known as germinants that signal favorable conditions for breaking dormancy through germination, an initial step in the process of completing the lifecycle by returning to vegetative bacteria. For example, the commercial microbial products may be designed to be dispersed into an environment where the spores encounter the germinants present in the environment to germinate into vegetative cells and perform an intended function. A variety of different bacteria may form spores. Bacteria from any of these groups may be used in the compositions, methods, and kits disclosed herein. For example, some bacteria of the following genera may form spores: *Acetonema, Alkalibacillus, Ammoniphilus, Amphibacillus, Anaerobacter, Anaerospora, Aneurinibacillus, Anoxybacillus, Bacillus, Brevibacillus, Caldanaerobacter , Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Filifactor, Filobacillus, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella,*

*Natroniella, Oceanobacillus, Orenia, Ornithinibacillus, Oxalophagus, Oxobacter, Paenibacillus, Paraliobacillus, Pelospora, Pelotomaculum, Piscibacillus, Planifilum, Pontibacillus, Propionispora, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus,* and/ or *Vulcanobacillus.*

Preferably, the bacteria that may form spores are from the family *Bacillaceae,* such as species of the genera *Aeribacillus, Aliibacillus, Alkalibacillus, Alkalicoccus, Alkalihalobacillus, Alkalilactibacillus, Allobacillus, Alteribacillus, Alteribacter,Amphibacillus, Anaerobacillus,Anoxybacillus,Aquibacillus, Aquisalibacillus, Aureibacillus, Bacillus, Caldalkalibacillus, Caldibacillus, Calditerricola, Calidifontibacillus, Camelliibacillus, Cerasibacillus, Compostibacillus, Cytobacillus, Desertibacillus, Domibacillus, Ectobacillus, Evansella, Falsibacillus, Ferdinandcohnia, Fermentibacillus, Fictibacillus, Filobacillus, Geobacillus, Geomicrobium, Gottfriedia, Gracilibacillus, Halalkalibacillus, Halobacillus, Halolactibacillus, Heyndrickxia, Hydrogenibacillus, Lederbergia, Lentibacillus, Litchfieldia, Lottiidibacillus, Margalitia, Marinococcus, Melghiribacillus, Mesobacillus, Metabacillus, Microaerobacter, Natribacillus, Natronobacillus, Neobacillus, Niallia, Oceanobacillus, Ornithinibacillus, Parageobacillus, Paraliobacillus, Paralkalibacillus, Paucisalibacillus, Pelagirhabdus, Peribacillus, Piscibacillus, Polygonibacillus, Pontibacillus, Pradoshia, Priestia, Pseudogracilibacillus, Pueribacillus, Radiobacillus, Robertmurraya, Rossellomorea, Saccharococcus, Salibacterium, Salimicrobium, Salinibacillus, Salipaludibacillus, Salirhabdus, Salisediminibacterium, Saliterribacillus, Salsuginibacillus, Sediminibacillus, Siminovitchia, Sinibacillus, Sinobaca, Streptohalobacillus, Sutcliffiella, Swionibacillus, Tenuibacillus, Tepidibacillus, Terribacillus, Terrilactibacillus, Texcoconibacillus, Thalassobacillus, Thalassorhabdus, Thermolongibacillus, Virgibacillus, Viridibacillu, Vulcanibacillus, Weizmannia.* In various examples, the bacteria may be strains of *Bacillus Bacillus acidicola, Bacillus aeolius, Bacillus aerius, Bacillus aerophilus, Bacillus albus, Bacillus altitudinis, Bacillus alveayuensis, Bacillus amyloliquefaciensex, Bacillus anthracis, Bacillus aquiflavi, Bacillus atrophaeus, Bacillus australimaris, Bacillus badius, Bacillus benzoevorans, Bacillus cabrialesii, Bacillus canaveralius, Bacillus capparidis, Bacillus carboniphilus, Bacillus cereus, Bacillus chungangensis, Bacillus coahuilensis, Bacillus cytotoxicus, Bacillus decisifrondis, Bacillus ectoiniformans, Bacillus enclensis, Bacillus fengqiuensis, Bacillus fungorum, Bacillus glycinifermentans, Bacillus gobiensis, Bacillus halotolerans, Bacillus haynesii, Bacillus horti, Bacillus inaquosorum, Bacillus infantis, Bacillus infernus, Bacillus isabeliae, Bacillus kexueae, Bacillus licheniformis, Bacillus luti, Bacillus manusensis, Bacillus marinisedimentorum, Bacillus mesophilus, Bacillus methanolicus, Bacillus mobilis, Bacillus mojavensis, Bacillus mycoides, Bacillus nakamurai, Bacillus ndiopicus, Bacillus nitratireducens, Bacillus oleivorans, Bacillus pacificus, Bacillus pakistanensis, Bacillus paralicheniformis, Bacillus paramycoides, Bacillus paranthracis, Bacillus pervagus, Bacillus piscicola, Bacillus proteolyticus, Bacillus pseudomycoides, Bacillus pumilus, Bacillus safensis, Bacillus salacetis, Bacillus salinus, Bacillus salitolerans, Bacillus seohaeanensis, Bacillus shivajii, Bacillus siamensis, Bacillus smithii, Bacillus solimangrovi, Bacillus songklensis, Bacillus sonorensis, Bacillus spizizenii, Bacillus spongiae, Bacillus stercoris, Bacillus stratosphericus, Bacillus subtilis, Bacillus swezeyi, Bacillus taeanensis, Bacillus tamaricis, Bacillus tequilensis, Bacillus thermocloacae, Bacillus thermotolerans, Bacillus thuringiensis, Bacillus tianshenii, Bacillus toyonensis, Bacillus tropicus, Bacillus vallismortis, Bacillus velezensis, Bacillus wiedmannii, Bacillus wudalianchiensis, Bacillus xiamenensis, Bacillus xiapuensis, Bacillus zhangzhouensis,* or combinations thereof.

In some examples, the bacterial strains that form spores may be strains of Bacillus, including: *Bacillus sp.* strain SD-6991; *Bacillus sp.* strain SD-6992; *Bacillus sp.* strain NRRL B-50606; *Bacillus sp.* strain NRRL B-50887; *Bacillus pumilus* strain NRRL B-50016; *Bacillus amyloliquefaciens* strain NRRL B-50017; *Bacillus amyloliquefaciens* strain PTA-7792 (previously classified as *Bacillus atrophaeus); Bacillus amyloliquefaciens* strain PTA-7543 (previously classified as *Bacillus atrophaeus*); *Bacillus amyloliquefaciens* strain NRRL B-50018; *Bacillus amyloliquefaciens* strain PTA-7541; *Bacillus amyloliquefaciens* strain PTA-7544; *Bacillus amyloliquefaciens* strain PTA-7545; *Bacillus amyloliquefaciens* strain PTA-7546; *Bacillus subtilis* strain PTA-7547; *Bacillus amyloliquefaciens* strain PTA-7549; *Bacillus amyloliquefaciens* strain PTA-7793; *Bacillus amyloliquefaciens* strain PTA-7790; *Bacillus amyloliquefaciens* strain PTA-7791; *Bacillus subtilis* strain NRRL B-50136 (also known as DA-33R, ATCC accession No. 55406); *Bacillus amyloliquefaciens* strain NRRL B-50141; *Bacillus amyloliquefaciens* strain NRRL B-50399; *Bacillus licheniformis* strain NRRL B-50014; *Bacillus licheniformis* strain NRRL B-50015; *Bacillus amyloliquefaciens* strain NRRL B-50607; *Bacillus subtilisstrain* NRRL B-50147 (also known as 300R); *Bacillus amyloliquefaciensstrain* NRRL B-50150; *Bacillus amyloliquefaciens* strain NRRL B-50154; *Bacillus megaterium* PTA-3142; *Bacillus amyloliquefaciens* strain ATCC accession No. 55405 (also known as 300); *Bacillus amyloliquefaciens* strain ATCC accession No. 55407 (also known as PMX); *Bacillus pumilus* NRRL B-50398 (also known as ATCC 700385, PMX-1, and NRRL B-50255); *Bacillus cereus* ATCC accession No. 700386; *Bacillus thuringiensis* ATCC accession No. 700387 (all of the above strains are available from Novozymes, Inc., USA); *Bacillus amyloliquefaciens* FZB24 (e.g., isolates NRRL B-50304 and NRRL B-50349 TAEGRO® from Novozymes), *Bacillus subtilis* (e.g., isolate NRRL B-21661 in RHAPSODY®, SERENADE® MAX and SERENADE® ASO from Bayer CropScience), *Bacillus pumilus* (e.g., isolate NRRL B-50349 from Bayer CropScience), *Bacillus*

*amyloliquefaciens* TrigoCor (also known as "TrigoCor 1448"; e.g., isolate Embrapa Trigo Accession No. 144/88.4Lev, Cornell Accession No.Pma007BR-97, and ATCC accession No. 202152, from Cornell University, USA) and combinations thereof.

In some examples, the bacterial strains that form spores may be strains of *Bacillus amyloliquefaciens.* For example, the strains may be *Bacillus amyloliquefaciens strain* PTA-7543 (previously classified as *Bacillus atrophaeus*), and/or *Bacillus amyloliquefaciens* strain NRRL B-50154, *Bacillus amyloliquefaciens* strain PTA-7543 (previously classified as *Bacillus atrophaeus*), *Bacillus amyloliquefaciens* strain NRRL B-50154, or from other *Bacillus amyloliquefaciens* organisms.

In some examples, the bacterial strains that form spores may be *Brevibacillus spp.,* e.g., *Brevibacillus brevis; Brevibacillus formosus*; *Brevibacillus laterosporus; or Brevibacillus parabrevis,* or combinations thereof.

In some examples, the bacterial strains that form spores may be *Paenibacillus spp.,* e.g., *Paenibacillus alvei; Paenibacillus amylolyticus; Paenibacillus azotofixans; Paenibacillus cookii; Paenibacillus macerans; Paenibacillus polymyxa; Paenibacillus validus,* or combinations thereof.

The bacterial spores may have an average particle diameter of about 2-50 microns, suitably about 10-45 microns. Bacillus spores are commercially available in blends in aqueous carriers and are insoluble in the aqueous carriers. Other commercially available bacillus spore blends include without limitation Freshen Free™ CAN (10X), available from Novozymes Biologicals, Inc.; Evogen® Renew Plus (10X), available from Genesis Biosciences, Inc.; and Evogen® GT (10X, 20X and 110X), all available from Genesis Biosciences, Inc. In the foregoing list, the parenthetical notations (10X, 20X, and 110X) indicate relative concentrations of the *Bacillus* spores.

Bacterial spores used in the compositions, methods, and uses disclosed herein may or may not be heat activated. In some examples, the bacterial spores are heat activated. In some examples, the bacterial spores are not heat inactivated. Preferably, the spores used herein are heat activated. Heat activation may comprise heating bacterial spores from room temperature (15- 25°C) to optimal temperature of between 25-120°C, preferably between 40C-100°C, and held the optimal temperature for not more than 2 hours, preferably between 70-80°C for 30 min.

For the methods, compositions and uses disclosed herein, populations of bacterial spores are generally used. In some examples, a population of bacterial spores may include bacterial spores from a single strain of bacterium. Preferably, a population of bacterial spores may include bacterial spores from 2, 3, 4, 5, or more strains of bacteria. Generally, a population of bacterial spores contains a majority of spores and a minority of vegetative cells. In some examples, a population of bacterial spores does not contain vegetative cells. In some examples, a population of bacterial spores may contain less than about 1%, 2%, 3%, 4%, 5%, 6%, 7%, 8%, 9%, 10%, 15%, 20%, 25%, 30%, 40%, or 50% vegetative cells, where the percentage of bacterial spores is calculated as ((vegetative cells/ (spores in population + vegetative cells in population)) $\times$ 100). Generally, populations of bacterial spores used in the disclosed methods, compositions and products are stable (i.e. not undergoing germination), with at least some individual spores in the population capable of germinating.

Populations of bacterial spores used in this disclosure may contain bacterial spores at different concentrations. In various examples, populations of bacterial spores may contain, without limitation, at least $1\times10^2$, $5\times10^2$, $1\times10^3$, $5\times10^3$, $1\times10^4$, $5\times10^4$, $1\times10^5$, $5\times10^5$, $1\times10^6$, $5\times10^6$, $1\times10^7$, $5\times10^7$, $1\times10^8$, $5\times10^8$, $1\times10^9$ $5\times10^9$, $1\times10^{10}$, $5\times10^{10}$, $1\times10^{11}$, $5\times10^{11}$, $1\times10^{12}$, $5\times10^{12}$, $1\times10^{13}$, $5\times10^{13}$, $1\times10^{14}$, or $5\times10^{14}$ spores/ml, spores/gram, or spores/cm3.

The composition comprises optional cleaning adjunct. Typically, the cleaning adjunct will be present in the composition in an amount from 1 to 98.9 wt%, more typically from 5 to 90 wt% cleaning adjunct. Suitable cleaning adjuncts comprise: additional surfactants, builders, bleach ingredients, colorants, chelating agents, dye transfer agents, deposition aids, dispersants, additional enzymes, and enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof. The composition may be such that the cleaning adjunct comprises one or more selected from the group consisting of (i) perfume microcapsule; (ii) fabric hueing agent; (iii) protease; (iv) amphiphilic cleaning polymer; (v) lipase, or (vi) mixtures thereof. Anionic Surfactant

The present inventors have found that the enzyme combination provides good soil breakdown, however the removal of the products of the breakdown of the substrates and soils containing them is improved by the presence of anionic surfactant. Therefore, the laundry detergent composition comprises from 1 to 60 wt% an anionic surfactant. Preferably the weight ratio of anionic surfactant to active alginate lyase enzyme protein is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1. Preferably the weight ratio of anionic surfactant to active booster enzyme protein is at least 500:1, preferably at least 1000:1 or at least 1500:1 or at least 2000:1, preferably being no greater than 500000:1, preferably no greater than 400000:1, or no greater than 200000:1 or up to 150000:1 or 100000:1, or 50000:1 or 10000:1.

Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof are also suitable anionic surfactants.

The anionic surfactant is preferably added to the detergent composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or triethanolamine. The composition preferably comprises from 1 to 60 weight % or from 1 to 50 wt% or 2 or 5 to 40 wt% of the composition, anionic surfactant. The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The invention also provides a cleaning composition comprising: from 0.00005 to 5 wt% (active enzyme protein) of an alginate lyase enzyme; 0.00005 to 5wt% booster enzyme (active enzyme protein) and a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ™, Lutensol™ and Pluronic™ from BASF, Dehypon™ series from Cognis and Genapol™ series from Clariant.

The detergent composition preferably comprises from 0.5wt% to about 40wt% of a non-ionic surfactant, preferably 1 to 30 wt% of the composition non-ionic surfactant.

The detergent composition preferably comprises one or more additional enzymes. Therefore, a preferred composition comprises (a) alginate lyase and (b) one or more additional enzymes selected from the group consisting of aminopeptidase, amylase, carbohydrase, carboxypeptidase, catalase, cellulase, chitinase, cutinase, cyclodextrin glycosyltransferase, esterase, alpha-galactosidase, beta-galactosidase, glucoamylase, alpha-glucosidase, beta-glucosidase, haloperoxidase, hexosaminidase, invertase, laccase, lipase, mannanase, mannosidase, nuclease, oxidase, pectinolytic enzyme, peptidoglutaminase, peroxidase, phytase, polyphenoloxidase, proteolytic enzyme, transglutaminase, xylanase, xanthan lyase, xanthanase, endo-β-1,3-glucanase and mixtures thereof. Preferably the cleaning composition comprises additional enzyme selected from nuclease, such as DNase or RNase, mannanase, xanthan lyase, xanthanase, amylase and mixtures thereof.

Preferably the composition comprises additional enzymes selected from xanthan lyase, xanthanase, mannanase and mixtures thereof. Mannanase is particularly preferred.

Preferably the composition comprises a protease or mixture of more than one protease, a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Preferably, the product of the invention comprises at least 0.01 mg, preferably from about 0.05 to about 10, more preferably from about 0.1 to about 6, especially from about 0.2 to about 5 mg of active further enzyme/ g of composition.

Preferably, the composition of the invention comprises a protease and an amylase and optionally a lipase.

If present, levels of protease in the composition of the invention include from about 0.05 to about 10, more preferably from about 0.5 to about 7 and especially from about 1 to about 6 mg of active protease/g of composition. Preferably, the composition comprises at least 0.01 mg, preferably from about 0.05 to about 10, more preferably from about 0.1 to about 6, especially from about 0.2 to about 5 mg of active amylase/ g of composition.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 or EP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint® Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenyl-methane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites, useable in laundry preferably has the formula $Na_{12}(AlO_2)_{12}(SiO_2)_{12} \cdot 27H_2O$ and the particle size is usually between 1-10 $\mu$m for zeolite A and 0.7-2 um for zeolite MAP. Other builders are Sodium metasilicate ($Na_2SiO_3 \cdot nH_2O$ or $Na_2Si_2O_5 \cdot n H_2O$) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.

Amphiphilic cleaning polymers: Preferably, the amphiphilic cleanimg polymer is a compound having the following general structure: bis(($C_2H_5O$)($C_2H_4O$)n)($CH_3$)-$N^+$-$C_xH_{2x}$-$N^+$-($CH_3$)-bis(($C_2H_5O$)($C_2H_4O$)n), wherein n = from 20 to 30, and x = from 3 to 8, or sulphated or sulphonated variants thereof.

Amphiphilic alkoxylated grease cleaning polymers of the present invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers having a structure as defined by one of the following structures (I), (II) or (III):

(I)

$$-[(OCHR^1-CHR^2)_a-O-OC-Ar-CO-]_d$$

(II)

$$-[(OCHR^3-CHR^4)_b-O-OC-sAr-CO-]_e$$

(III)

$$-[(OCHR^5-CHR^6)_c-OR^7]_f$$

wherein:

a, b and c are from 1 to 200;
d, e and f are from 1 to 50;
Ar is a 1,4-substituted phenylene;
sAr is 1,3-substituted phenylene substituted in position 5 with $SO_3Me$;
Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
$R^1$, $R^2$, $R^3$, $R^4$, $R^5$ and $R^6$ are independently selected from H or $C_1$-$C_{18}$ n- or iso-alkyl; and
$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g. fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or

perfumes.

<u>Method of Use</u>

The present invention also provides a method for treating a fabric, the method comprising in a contacting step, contacting a fabric with an aqueous wash liquor comprising an alginate lyase enzyme as described above, preferably in an amount from 0.01ppm to 10ppm, preferably from 0.1ppm to 1ppm; and booster enzyme as described above preferably in an amount from 0.01ppm to 10ppm, preferably from 0.1ppm to 1ppm; and preferably additionally an anionic surfactant, preferably in an amount from 0.05 to 50g/l, more preferably from 0.2g/l to 5g/l or 0.5g/l to 3g/l.

The aqueous wash liquor may be formed by adding a composition as described above to water, for example in a washing machine or hand washing process. Alternatively, the aqueous wash liquor may be formed by adding the alginate lyase enzyme, booster enzyme and any cleaning adjunct as separate components, into water to form the wash liquor. The fabric may be optionally subsequently washed, and/or rinsed and/or dried.

The alginate lyase enzyme may be present in the wash liquor in an amount corresponding to 0.001-100 mg of active enzyme protein per liter of wash liquor, preferably 0.005-5 mg of active enzyme protein per liter of wash liquor, more preferably 0.01-1 mg of enzyme protein per liter of wash liquor and in particular 0.1-1 mg of enzyme protein per liter of wash.

In the contacting step, or in a subsequent step, it may be preferred to use mechanical agitation to promote cleaning and removal of the broken-down soil by-products from the fabric. The wash liquor preferably has a pH of from about 7 or 8 to about 10.5. The composition may typically be employed at concentrations of from about 500 ppm to about 15,000 ppm in solution to form the wash liquor. The wash liquor preferably has a temperature from about 5 °C to about 40 °C, or preferably from 10 to 35 °C or 30 °C or 25 °C. The water to fabric ratio is typically from about 1:1 to about 30:1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Example

The following testing measures the incremental stain removal benefit of adding a Bacillus spore consortium to a detergent composition, including the impact of including alginate lyase enzyme in the detergent composition.

Stain Preparation

For each of two tests, 32 Chocolate mousse stains were prepared by mixing 11g of Milk Chocolate mousse mix (Bird's brand, Premier Foods, St Albans, UK) with 26ml of long-life whole milk (Asda Stores Ltd, Leeds, UK). 0.4g of this mixture was pipetted onto 5cm $\times$ 5cm knitted cotton fabrics (GMT desized knitted cotton, Warwick Equest Ltd, Consett, UK) using a positive displacement pipette, and spread into the fabric within a circular stain template using the back of a sterile metal spoon, before being left to dry overnight in a biosafety cabinet.

For each test leg comprising Bacillus spores, dried mousse stains were pre-treated with 200µl of sterile deionized (DI) water and 40µl of a Bacillus spore suspension containing 1x108 CFU/ml Bacillus (prepared using Evozyme® P500 BS7 supplied by Genesis Biosciences, Cardiff, UK) in DI water. For test legs without Bacillus spores, dried chocolate mousse stains were pre-treated with 240µl DI water alone. The stain replicates of each pre-treatment leg were placed into separate 350ml sealed containers with one damp 5cm $\times$ 5cm knitted cotton swatch (100% DI water by weight), and stored for 72 hours at 21°C to simulate storage in a laundry hamper.

General washing protocol and stain removal analysis method

Each test involved four external and two internal replicates for each of the four treatments. i.e., Two chocolate mousse stains were included within each wash, with each leg repeated four times to give a total of eight replicates.

Treatments were as follows:

Reference
Reference + Bacillus spore pre-treatment
Reference + 2mg/L alginate lyase (Alginate Lyase S, supplied by Nagase Chemtex, Osaka, Japan)
Reference + Bacillus spore pre-treatment + 2mg/L alginate lyase

1.19g/ L of Tide liquid detergent (supplied by Procter & Gamble, Cincinnati, USA) was used as the reference detergent and dosed into each wash along with the treatments described above.

This reference detergent does not contain Bacillus spores or alginate lyase enzyme.

In the first test the following wash steps were conducted with hard water (20 USA grains per gallon). In the second test the same wash steps were conducted, but instead with medium hardness water (8 USA grains per gallon).

For both tests, the washing step was conducted in a 1L tergotometer. The fabric load in each comprised the two pre-treated chocolate mousse stains and enough 5cm × 5cm knitted cotton ballast (GMT desized knitted cotton, Warwick Equest Ltd, Consett, UK) to make the total load weight to 60g. The fabrics were washed for 17 minutes at 27°C, 208rpm, and then rinsed once for 5 minutes (15°C).

Stains were left to dry overnight and evaluated for stain removal using L*a*b* readings taken using a DigiEye (VeriVide Ltd, Leicester, UK) at shutter speed 1/2, radius 180 which was calibrated before use. L*a*b* measurements were taken for unwashed stains and washed stains, and Delta E* calculations made to determine the level of staining for both unwashed stains and washed stains compared to the unsoiled fabric using the following equation where the suffix 1 denotes the values for the unsoiled fabric and the suffix 2 denotes the values for the unwashed or washed stains.

The Stain Removal Index (SRI) is the level of stain removal calculated as a percentage as follows:

$$\text{SRI} = 100 \text{ x } (A - B) / A$$

Where:

A = Delta E* of Unwashed fabric-stained region
B = Delta E* of Washed fabric-stained region

SRI Data:

| Test 1: Hard Water (20gpg) | | |
|---|---|---|
| | SRI (Std Dev) | Benefit for addition of BS7 Delta SRI (Std Dev) |
| Reference | 36.16 (4.02) | +3.32 (2.73) |
| Reference + alginate lyase | 44.70 (3.95) | +4.45 (3.26) s |
| Test 2: City Water (8gpg) | | |
| | SRI (Std Dev) | Benefit for addition of BS7 Delta SRI (Std Dev) |
| Reference | 46.09 (1.79) | +1.01 (2.02) |
| Reference + alginate lyase | 55.97 (0.53) | +3.83 (2.92) s |
| *s highlights the stain removal benefit is statistically significant with 95% confidence level. *s highlights the stain removal benefit is statistically significant with 95% confidence level. | | |

Conclusion

The results show that addition of Bacillus spore delivers a statistically significant improvement in stain removal when added to a detergent composition comprising alginate lyase, whereas no significant improvement is observed when the Bacillus spore is added to the control detergent that does not contain alginate lyase. The same trend is observed under both hardness conditions. This result is surprising and unexpected, because the detergent comprising alginate lyase already achieves a much higher level of removal than the reference detergent without this enzyme and therefore has much more limited scope for incremental stain removal improvement. The results suggest a surprising complementary benefit profile for these ingredients, possibly because the Bacillus spores are producing enzymes that are complementary to the formulated alginate lyase in the inventive composition.

**Claims**

1. A detergent composition, preferably a laundry detergent composition, comprising (a) from 0.00005 to 5 wt% alginate lyase enzyme (active enzyme protein) and (b) *Bacillus* spores.

**2.** A detergent composition according to claim 1 wherein the alginate lyase enzyme is of microbial origin, preferably bacterial or algal, most preferably bacterial.

**3.** A detergent composition according to claim 1 or claim 2 wherein the alginate lyase enzyme is obtainable from *Flavobacterium sp, Sphingomonas sp, Zobellia galactanivorans*, preferably *Flavobacterium sp.*

**4.** A detergent composition according to any preceding claim wherein the alginate lyase enzyme comprises: an alginate lyase selected from alginate lyase having at least 60%, or at least 70%, or at least 80%, or at least 90%, or at least 95%, or at least 96%, or at least 97%, or at least 98%, or at least 99%, or 100%, sequence identity to one or more of SEQ ID NO: 1; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 2; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 3; an alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 4; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 5; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 6; alginate lyase having at least 60%, or at least 70%, or at least 80%, or at least 90%, or at least 95%, or at least 96%, or at least 97%, or at least 98%, or at least 99%, or 100%, sequence identity to SEQ ID NO: 7, and mixtures thereof, preferably wherein the alginate lyase enzyme comprises: an alginate lyase selected from alginate lyase having at least 60% sequence identity to one or more of SEQ ID NO: 1; SEQ ID NO: 5; SEQ ID NO: 6; and SEQ ID NO: 7, or mixtures thereof, most preferably wherein the alginate lyase enzyme comprises: an alginate lyase selected from alginate lyase having at least 60%, sequence identity to one or more of SEQ ID NO: 6; and SEQ ID NO: 7 and mixtures thereof.

**5.** A detergent composition according to any preceding claim wherein the *Bacillus* spores are selected from the group consisting of *Bacillus subtilis, Bacillus amyloliquefaciens, Bacillus licheniformis, Bacillus megaterium, Bacillus pumilus, Bacillus cereus, Bacillus thuringiensis, Bacillus mycoides, Bacillus tequilensis, Bacillus vallismortis, Bacillus mojavensis* and mixtures thereof, preferably selected from the group consisting of *Bacillus subtilis, Bacillus amyloliquefaciens, Bacillus licheniformis, Bacillus megaterium, Bacillus pumilus* and mixtures thereof.

**6.** A detergent composition according to any preceding claim additionally comprising surfactant, preferably wherein the surfactant is present in an amount such that the weight ratio of surfactant to active alginate lyase enzyme protein is at least 500:1, preferably at least 1000:1.

**7.** A detergent composition according to any preceding claim additionally comprising an enzyme selected from amylase, protease, nuclease enzyme, hexosaminidase enzyme, endo-β-1,3-glucanase enzyme, and mixtures thereof, the or each enzyme being present in an amount from 0.00005 to 5 wt% active enzyme protein.

**8.** A detergent composition according to any preceding claim wherein the composition additionally comprises nonionic surfactant, preferably in an amount from 1 to 30 wt% of the composition.

**9.** A detergent composition according to any preceding claim wherein the composition additionally comprises surfactant, said surfactant comprising an anionic and a nonionic surfactant, preferably in a weight ratio of anionic to nonionic surfactant of from 30:1 to 1:2, preferably from 20:1 to 2:3, or to 1:1.

**10.** A method of treating a fabric, the method comprising contacting a fabric with an aqueous wash liquor comprising (a) an alginate lyase enzyme; (b) *Bacillus* spores; and (iii) optionally a cleaning adjunct.

**11.** Use of a composition or method according to any of claims 1 to 10 for improving fabric stain removal.

**12.** Use according to the preceding claim for the removal of sugary and/or fatty stain containing protein.

Europäisches
Patentamt

European
Patent Office

Office européen
des brevets

# EUROPEAN SEARCH REPORT

**Application Number**

EP 23 18 0609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/009922 A1 (KLINGMAN SHANNON E [US] ET AL) 14 January 2021 (2021-01-14) | 10 | INV. C11D3/38 |
| Y | * paragraphs [0002], [0003], [0030], [0031], [0037] – [0039], [0041], [0051] – [0053], [0100] – [0102], [0109], [0128], [0129]; claims * <br> * paragraphs [0137] – [0141], [0173]; figure 6 * <br> * claims; examples * | 1-9,11 | C11D3/386 C11D11/00 C12N9/88 |
| Y | WO 2022/094590 A1 (PROCTER & GAMBLE [US]) 5 May 2022 (2022-05-05) <br> * page 1, paragraph 1 – page 4, paragraph 1 * <br> * page 5, paragraph 2-3; examples * <br> * page 7, paragraph 1 – page 10, paragraph 24 * | 1-12 | |
| Y | US 2023/023684 A1 (LANT NEIL JOSEPH [GB] ET AL) 26 January 2023 (2023-01-26) <br> * paragraphs [0002], [0005] – [0009], [0015] – [0023], [0035] – [0037], [0044]; claims; examples * | 1-12 | |

**TECHNICAL FIELDS SEARCHED (IPC)**

C11D
C12N

The present search report has been drawn up for all claims

| Place of search | Date of completion of the search | Examiner |
|---|---|---|
| The Hague | 13 November 2023 | Marttin, Emmeline |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

......................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 18 0609

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2023

| Patent document cited in search report | Publication date | Patent family member(s) | | Publication date |
|---|---|---|---|---|
| US 2021009922 A1 | 14-01-2021 | NONE | | |
| WO 2022094590 A1 | 05-05-2022 | CA | 3196356 A1 | 05-05-2022 |
| | | CA | 3196361 A1 | 05-05-2022 |
| | | CA | 3196363 A1 | 05-05-2022 |
| | | CA | 3196371 A1 | 05-05-2022 |
| | | CA | 3196497 A1 | 05-05-2022 |
| | | CN | 116323936 A | 23-06-2023 |
| | | CN | 116348580 A | 27-06-2023 |
| | | CN | 116348581 A | 27-06-2023 |
| | | CN | 116391035 A | 04-07-2023 |
| | | CN | 116391036 A | 04-07-2023 |
| | | EP | 4237526 A1 | 06-09-2023 |
| | | EP | 4237527 A1 | 06-09-2023 |
| | | EP | 4237528 A1 | 06-09-2023 |
| | | EP | 4237529 A1 | 06-09-2023 |
| | | EP | 4237530 A1 | 06-09-2023 |
| | | JP | 2023543060 A | 12-10-2023 |
| | | JP | 2023543893 A | 18-10-2023 |
| | | JP | 2023547171 A | 09-11-2023 |
| | | US | 2023250367 A1 | 10-08-2023 |
| | | US | 2023279315 A1 | 07-09-2023 |
| | | US | 2023279316 A1 | 07-09-2023 |
| | | US | 2023313075 A1 | 05-10-2023 |
| | | US | 2023340378 A1 | 26-10-2023 |
| | | WO | 2022094163 A1 | 05-05-2022 |
| | | WO | 2022094164 A1 | 05-05-2022 |
| | | WO | 2022094588 A1 | 05-05-2022 |
| | | WO | 2022094589 A1 | 05-05-2022 |
| | | WO | 2022094590 A1 | 05-05-2022 |
| US 2023023684 A1 | 26-01-2023 | EP | 4123008 A1 | 25-01-2023 |
| | | US | 2023023684 A1 | 26-01-2023 |
| | | WO | 2023004214 A2 | 26-01-2023 |

EPO FORM P0459

For more details about this annex : see Official Journal of the European Patent Office, No. 12/82

EP 4 481 026 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1794275 A
- EP 1794276 A
- US 7208459 B2
- WO 201198355 A
- WO 201147987 A
- US 2012090102 A
- WO 2010145887 A

- WO 2006055787 A
- WO 2010142503 A
- US 20080177090 A
- WO 2011011799 A
- US 8138222 B
- WO 09118375 A
- WO 9813459 A

**Non-patent literature cited in the description**

- **NEEDLEMAN** ; **WUNSCH**. *J. Mol. Biol.*, 1970, vol. 48, 443-453

- **RICE et al.** EMBOSS: The European Molecular Biology Open Software Suite. *Trends Genet.*, 2000, vol. 16, 276-277

16